Europäisches Patentamt

European Patent Office

Office européen des brevets

(11) Publication number: **0 148 584**
A2

## (12) EUROPEAN PATENT APPLICATION

(21) Application number: 84308272.8

(22) Date of filing: 29.11.84

(51) Int. Cl.⁴: **C 07 C 15/02,** C 07 C 2/66,
C 07 C 39/08, C 07 C 37/08

(30) Priority: 04.01.84 US 568012
04.01.84 US 568011

(43) Date of publication of application: **17.07.85**
**Bulletin 85/29**

(84) Designated Contracting States: **BE DE FR GB IT NL**

(71) Applicant: **MOBIL OIL CORPORATION, 150 East 42nd
Street, New York New York 10017 (US)**

(72) Inventor: **Kaeding, Warren William, 6 Roseberry Court
Lawrenceville, New Jersey 08648 (US)**

(74) Representative: **West, Alan Harry, Mobil
Court 3 Clements Inn, London WC2A 2EB (GB)**

(54) Production of para-diisopropylbenzene and use of same in the production of hydroquinone.

㊾ A process is disclosed whereby cumene and/or benzene is brought into contact with propylene in the presence of a ZSM-12 catalyst to selectively produce para-diisopropylbenzene. The para-diisopropylbenzene product may then be oxidized to the corresponding dihydroperoxide followed by rearrangement to produce hydroquinone and acetone.

## PRODUCTION OF PARA-DIISOPROPYLBENZENE
## AND USE OF SAME IN THE PRODUCTION OF
## HYDROQUINONE

This invention relates to a process for the selective production of para-diisopropylbenzene by catalytic propylation of cumene and/or benzene and to the conversion of the resultant para-diisopropylbenzene to hydroquinone.

Both para-diisopropylbenzene and hydroquinone have a number of commercial uses; para-diisopropylbenzene as a solvent and chemical intermediate and hydroquinone in photographic developers, medicines, dye intermediates, anti-oxidants, inhibiters and stabilizers.

Alkylation of aromatic hydrocarbons utilizing crystalline zeolite catalysts has heretofore been described. For example, U.S. Pat. No. 2,904,607 to Mattox refers to alkylation of aromatic hydrocarbons with an olefin in the presence of a crystalline metallic aluminosilicate having uniform pore openings of about 6 to 15 Angstrom unit. U.S. Pat. No. 3,251,897 describes alkylation of aromatic hydrocarbons in the presence of X- or Y-type crystalline zeolites, specifically such type zeolites wherein the cation is rare earth and/or hydrogen. U.S. Pat. No. 3,751,504 and U.S. Pat. No. 3,751,506 describe vapor phase alkylation of aromatic hydrocarbons with olefins in the presence of a ZSM-5 type of zeolite catalyst.

U.S. Pat. No. 3,755,483 discloses vapor phase alkylation of aromatic hydrocarbons in the presence of a ZSM-12 zeolite catalyst. The reaction is carried out at temperatures between the critical temperature of the aromatic compound and 482°C (900°F). The critical temperature and pressure of benzene are 288.9°C (552°F) and 48.6 atm. ($4.9 \times 10^6$ N/m$^2$).

Harper et al have described the catalytic alkylation of benzene with propylene over a crystalline zeolite (Petrochemical Preprints, American Chemical Society, Vol. 22, No. 3, p. 1084, 1977). Extensive kinetic and catalyst aging studies were conducted with a rare earth-exchanged Y-type zeolite (REY) catalyst.

U.S. Pat. No. 4,393,262 describes a process whereby benzene is brought in contact with ZSM-12 to selectively produce isopropylbenzene (i.e. cumene). This process may be carried out in the liquid or the vapor phase at temperatures of from 100°C to 300°C and pressure ranging from $10^5$ N/m$^2$ to $6 \times 10^6$ N/m$^2$.

In accordance with one aspect of the present invention, there is provided a process for the selective propylation of cumene to produce para-diisopropylbenzene, said process comprising contacting a mixture of cumene and propylene with a ZSM-12 catalyst under propylation conditions.

In accordance with a further aspect of the invention, there is provided a process for the selective production of para-diisopropylbenzene, said process comprising the steps of:

- (i) contacting mixtures of benzene and propylene with a ZSM-12 catalyst under propylation conditions to selectively produce cumene; and
- (ii) contacting mixtures of said cumene of step (i) and propylene with a ZSM-12 catalyst under propylation conditions.

In accordance with another aspect of the invention, there is provided a process for the propylation of benzene with selective production of para-diisopropylbenzene, said process comprising contacting mixtures of benzene and propylene with a ZSM-12 catalyst under sufficient propylation conditions wherein the molar ratio of propylene to benzene is at least 2.

Conveniently, the resultant para-diisopropylbenzene is oxidized to the corresponding dihydroferoxide and the dihydroperoxide is rearranged to produce hydroquinone and acetone.

The crystalline zeolite utilized herein is ZSM-12, the characteristics and synthesis of which are described in, for example, U.S. Patent No. 3,832,449 and EP-B-18089.

When prepared in the presence of organic cations, ZSM-12 is substantially catalytically inactive, possibly because the intracrystalline free space is occupied by organic cations from the forming solution. It may be activated by heating in an inert atmosphere at 540°C for one hour, for example, followed by base exchange with ammonium salts followed by calcination at 540°C in air. The presence of organic cations in the forming solution may not be absolutely essential to the formation of the zeolite. More generally, it is desirable to activate this type of catalyst by base exchange with ammonium salts followed by calcination in air at 540°C for from 15 minutes to 24 hours.

When synthesized in the alkali metal form, the zeolite is conveniently converted to the hydrogen form, generally by intermediate formation of the ammonium form as a result of ammonium ion exchange and calcination of the ammonium form to yield the hydrogen form. In addition to the hydrogen form, other forms of the zeolite wherein the original alkali metal has been reduced to less than about 1.5 percent by weight may be used. Thus, the original alkali metal of the zeolite may be replaced by ion exchange with other suitable ions of Groups IB to VIII of the Periodic Table, including, by way of example, nickel, copper, zinc, palladium, calcium or rare earth metals.

In practicing the desired alkylation process, it may be desirable to incorporate the above described crystalline zeolite in another material resistant to the temperature and other conditions employed in the process. Such matrix materials include synthetic or naturally occurring substances as well as inorganic materials such as clay, silica and/or metal oxides. The latter may be either naturally occurring or in the form of gelatinous precipitates or gels including mixtures of silica and metal oxides. Naturally occurring clays which can be composited with the zeolite include those of the montmorillonite and kaolin families, which families include the sub-bentonites and the kaolins commonly known as Dixie, McNamee-Georgia and Florida clays or others in which the main mineral constituent is halloysite, kaolinite, dickite, nacrite or anauxite.  Such clays can be used in the raw state as originally mined or initially subjected to calcination, acid treatment or chemical modification.

In addition to the foregoing materials, the zeolite employed herein may be composited with a porous matrix material, such as alumina, silica-alumina, silica-magnesia, silica-zirconia, silica-thoria, silica-beryllia, silica-titania as well as ternary compositions, such as silica-alumina-thoria, silica-alumina-zirconia, silica-alumina-magnesia and silica-magnesia-zirconia.  The matrix may be in the form of a cogel.  The relative proportions of zeolite component and inorganic oxide gel matrix on an anhydrous basis may vary widely with the zeolite content ranging from between 1 to 99 percent by weight and more usually in the range of 5 to 80 percent by weight of the dry composite.

A second optional component of the ZSM-12 catalyst comprises a minor proportion, e.g., from 0.05% to 50% by weight of the catalyst composite, of a difficultly reducible oxide.  Oxides of this type can include oxides of phosphorus as well as those oxides of the metals of Groups IA, IIA, IIIA, IVA, VA, VIA, VIIA, VIIIA, IB, IIB, IIIB, IVB, or VB of the Periodic Chart of the Elements (Fisher Scientic Company, Catalog No. 5-702-10) which serve to enhance the para-selectivity properties of the catalysts modified therewith.  The difficultly reducible oxides most commonly employed to modify the selectivity properties of the catalyst herein are oxides of phosphorus and magnesium.  Thus, the catalyst can be treated with phosphorus and/or magnesium compounds in the manner described in U.S. Patent Nos.  3,894,104; 4,049,573; 4,086,287; and 4,128,592.

Phosphorus, for example, can be incorporated into the catalyst at least in part in the form of phosphorus oxide in an amount of from 0.25% to 25% by weight of the catalyst composition, preferably from 0.7% to 15% by weight.  Such incorporation can be readily effected by contacting the zeolite composite with a solution of an appropriate phosphorus compound, followed by drying and

F-2628 (2629)                    -5-                    0148584

calcining to convert phosphorus in the zeolite to its oxide form. Preferred phosphorus-containing compounds include diphenyl phosphine chloride, trimethylphosphite and phosphorus trichloride, phosphoric acid, phenyl phosphine oxychloride, trimethylphosphate, diphenyl phosphinous acid, diphenyl phosphinic acid, diethylchlorothiophosphate, methyl acid phosphate and other alcohol-$P_2O_5$ reaction products.  Particularly preferred are ammonium phosphates, including ammonium hydrogen phosphate, $(NH_4)_2HPO_4$, and ammonium dihydrogen phosphate, $NH_4H_2PO_4$.  Calcination is generally conducted in the presence of oxygen at a temperature of at least about 150°C.  However, higher temperatures, i.e., up to 500°C or higher are preferred.  Such heating is generally carried out for 3-5 hours but may be extended to 24 hours or longer.

Magnesium oxide is another preferred difficultly reducible oxide which can be incorporated with the zeolite composite in a manner similar to that employed with phosphorus.  Magnesium can comprise from 0.25% to 25% by weight preferably from 1% to 15% by weight present at least in part as magnesium oxide.  As with phosphorus, magnesium oxide incorporation is effected by contacting the zeolite composite with an appropriate magnesium compound followed by drying and calcining to convert magnesium in the zeolite to its oxide form.  Preferred magnesium-containing compounds include magnesium nitrate and magnesium acetate.  Calcination times and temperatures are generally the same as recited hereinbefore for calcination of the phosphorus-containing catalyst.

In addition to treatment of the zeolite to incorporate phosphorus and/or magnesium oxides, the zeolite may also be modified in a substantially similar manner to incorporate thereon a variety of other oxide materials to enhance para-selectivity.  Such oxide materials include oxides of boron (U.S. 4,067,920); antimony (U.S. 3,979,472); beryllium (U.S. 4,260,843); Group VIIA metals (U.S. 4,275,256); alkaline earth metals (U.S. 4,288,647); Group IB metals (U.S. 4,276,438); Group IVB metals (U.S. 4,278,827); Group VIA metals (U.S. 4,259,537); Group IA elements (U.S. 4,329,533); cadmium (U.S. 4,384,155); iron and/or cobalt (U.S. 4,380,685); Group IIIB metals (U.S. 4,276,437); Group IVA metals (U.S. 4,302,620); Group VA metals (U.S. 4,302,621); and Group IIIA elements (U.S. 4,302,622).

Cumene may be produced by the propylation of benzene in the presence of the ZSM-12 catalyst by contact of the benzene with propylene at a temperature between 100°C and 300°C, preferably between 150°C and 250°C. The reaction generally takes place at atmospheric pressure, but the pressure may be within the range of $10^5$ N/m$^2$ to 6 X $10^6$ N/m$^2$ (1 atm to 60 atm). The molar ratio of benzene to propylene is preferably within the range of 12:1 to 1:1. The reaction may be suitably accomplished utilizing a feed weight hourly space velocity (WHSV) of between 0.5 and 100, preferably between 5 and 40.

Propylation of cumene to produce para- diisopropylbenzene may be carried out under conditions analogous to those for producing cumene. More particularly, para-diisopropylbenzene may be produced by the propylation of cumene in the presence of the above-described catalyst by contact of the cumene with propylene at a temperature between 100°C and 300°C, and preferably between 150°C and 250°C. The reaction generally takes place at atmospheric pressure, but the pressure may be within the range of $10^5$ N/m$^2$ to 6 X $10^6$ N/m$^2$ (1 atm to 60 atm). The molar ratio of cumene to propylene is preferably within the range of 12:1 to 1:1. The reaction may be suitably accomplished utilizing a feed weight hourly space velocity (WHSV) of between 0.5 and 100, preferably between 5 and 40.

By means of the present process, it is possible to obtain a diisopropylbenzene product having, e.g., at least 80 of the para-isomer and, e.g., less than 1 percent of the ortho-isomer.

The process of this invention may be conducted with the organic reactants in either the gaseous or the liquid phase or both. It may be carried out as a batch-type, semi-continuous or continuous operation utilizing a fixed, fluidized or moving bed catalyst system. The Examples which follow will serve to illustrate the process of this invention.

When Alpha Value is mentioned in these Examples, it is noted that the Alpha Value is an approximate indication of the catalytic cracking activity of the catalyst compared to a standard catalyst and it gives the relative rate constant (rate or normal hexane conversion per volume of catalyst per unit time). It is based on the activity of the highly active silica-alumina cracking catalyst taken as an Alpha Value of 1 (Rate Constant = 0.016 sec$^{-1}$). The Alpha Test is described in U.S. Patent 3,354,078 and in The Journal of Catalysis, Vol. IV, pp. 522-529 (August 1965).

In the Examples which follow, the catalytic, vapor phase alkylation unit was constructed entirely of 316 or 321 stainless steel. The reactor dimensions were 1-1/4 inch (3.2 cm) outside diameter, 5/8 inch (1.6 cm) inside diameter and 21-5/8 inches (55 cm) long and rated to operate at 5000 psig (34576kPa) at 650°F (343°C). It was fitted with a metal, spiral insert in the top section which functioned as a preheater. The catalyst section contained a 1/8 inch (0.3 cm)centered thermowell. A three zone furnace with independent controls was used to obtain the desired temperature. Six or nine grams, 12-18 cc's, of the catalyst was centered in the middle zone with low surface area quartz chips to prevent void spaces.

Standard equipment such as pumps, temperature, pressure and flow control devices, feed and storage tanks were selected and designed to operate continuously and unattended for at least 24-hour periods. The propylene was stored in the liquid phase in a Jergeson gauge. Accurate metering to the reactor was supplied by two Isco pumps which could be operated individually or together. This provided flexibility to operate continuously for 24 hours and permit uninterrupted flow while the pump cylinders were refilled. Automatic detector and shut-off devices were installed in the event of a power failure. The entire unit was located in a large hood.

The liquid product was condensed with water cooling. The remaining gas was collected in a dry gas collecting tower with a mercury seal float. Liquid and gaseous compositions were analyzed by standard chromatography techniques. A 1/8" x 12' (0.3 x 366 cm) column containing 60-80 mesh silica gel, was used for the gas analysis.  A 150' (4572 cm) x 0.01" (0.03 cm) outside diameter Perkin-Elmer, OS-138-polyphenyl ether capillary column was used for analysis of the liquid product.  In order to determine the ortho/meta/para ratio of diisopropylbenzene (DIPB) product, a 50 meter, Chrompac fused silica column coated with CP-Wax CB was used for the liquid product.

Each of the ZSM-12 catalysts of the Examples which follow were intimately mixed with 35 wt.% alumina binder, then pressed into wafers, crushed and screened to a uniform particle size of 14-20 mesh.

## EXAMPLE 1

The ZSM-12 catalyst employed in this Example was a hydrogen form of ZSM-12 (i.e. HZSM-12) having a silica to alumina ratio of 213 and an Alpha Value of 39.  The zeolite was calcined in $N_2$ in the absence of binder, $NH_4^+$ ion exchanged then compounded with alumina and extruded, followed by drying and final calcination.

Conditions of reaction and a summary of analytical results are shown in Table 1 wherein "CU" stand for cumene, "NPRBZ" stands for n-propylbenzene and "Other" represents primarily propylene oligomers.  The ratio of para/meta DIPB isomers produced ranged from 76/23 at 175°C to 47/53 at 250°C.

## Table 1

Alkylation of Cumene with Propylene to Produce Diisopropylbenzene (at atmospheric pressure)

| Run No. | 1 | 2 | 3 | 4 | 5 | 6 | 7 | 8 |
|---|---|---|---|---|---|---|---|---|
| Temperature, °C | 175 | 200 | 225 | 250 | 175 | 200 | 225 | 250 |
| $CU/C_3H_6$ | | | | | | | | |
| WHSV | 6.8/.87 | 7.6/.87 | 7.7/.87 | 6.71/.87 | 14.3/.85 | 14.5/.85 | 14.9/.85 | 13.8/.85 |
| mole | 2.7/1 | 3.1/1 | 3.1/1 | 2.7/1 | 5.9/1 | 6.0/1 | 6.2/1 | 5.7/1 |
| **Conversion, wt%** | | | | | | | | |
| CU | 18 | 26 | 29 | 36 | 8 | 13 | 15 | 19 |
| $C_3H_6$ | 68 | 70 | 82 | 89 | 51 | 65 | 79 | 85 |
| **Selectivity, %** | | | | | | | | |
| Benzene | 2.2 | 1.2 | 1.3 | 3.3 | .9 | .7 | 1.7 | 4.8 |
| DIPB | | | | | | | | |
| para | 69.6 | 68.6 | 62.3 | 42.6 | 72.1 | 71.7 | 63.0 | 47.4 |
| meta | 21.0 | 23.1 | 31.3 | 47.8 | 22.9 | 23.9 | 32.0 | 44.0 |
| ortho | 1.4 | .9 | .4 | .3 | 1.4 | .8 | .4 | .3 |
| Total | 92.0 | 92.6 | 94.0 | 90.7 | 96.4 | 96.4 | 95.4 | 91.7 |
| NPRBZ | 0 | 0 | 0 | 0 | 0 | 0 | 0 | 0 |
| Other | 5.8 | 6.2 | 4.7 | 6.0 | 2.7 | 2.9 | 2.9 | 3.5 |
| DIPB % | | | | | | | | |
| para | 75.7 | 74.1 | 66.3 | 47.0 | 75.4 | 74.3 | 66.0 | 51.7 |
| meta | 22.8 | 29.4 | 33.3 | 52.7 | 23.1 | 24.8 | 33.6 | 48.0 |
| ortho | 1.5 | 1.0 | .4 | .3 | 1.5 | .8 | .4 | .3 |

## EXAMPLE 2

The HZSM-12 catalyst of this Example was essentially equivalent to that of Example 1 except that it was prepared in a slightly different manner. More particularly, the same zeolite as Example 1 was processed by the sequence of extruding, calcining, $NH_4^+$ exchange, drying and final calcination. The composition of the catalyst was the same as the composition of the catalyst of Example 1.

Conditions of reaction and a summary of analytical results are shown in Table 2, wherein "CU" stands for cumene, "NPRBZ" stands for n-propylbenzene and "Other" represents primarily propylene oligomers.

Table 2

Alkylation of Cumene with Propylene to Produce Diisopropylbenzene
(at atmospheric pressure)

| Run No. | 1 | 2 | 3 | 4 | 5 | 6 |
|---|---|---|---|---|---|---|
| Temp., °C | 150 | 200 | 250 | 300 | 250 | 300 |
| $CU/C_3H_6$ | | | | | | |
| WHSV | 7.2/.9 | 7.2/.9 | 7.2/.9 | 7.2/.9 | 13.9/1.7 | 13.9/1.7 |
| Mole | 2.9/1 | 2.9/1 | 2.9/1 | 2.9/1 | 2.8/1 | 2.8/1 |
| **Conversion, wt%** | | | | | | |
| CU | 13.4 | 17.2 | 26.9 | 40.7 | 16.6 | 33.9 |
| $C_3H_6$ | 27.5 | 48.0 | 76.7 | 47.0 | 45.3 | 50.8 |
| **Selectivity, wt%** | | | | | | |
| Benzene | 11.0 | 1.5 | 4.3 | 25.4 | 3.8 | 17.9 |
| DIPB | | | | | | |
| para | 67.4 | 74.2 | 49.1 | 20.0 | 66.2 | 27.5 |
| meta | 18.6 | 20.7 | 41.1 | 40.8 | 25.3 | 45.6 |
| ortho | .9 | .5 | .2 | .2 | .15 | .1 |
| Total | 86.9 | 95.4 | 90.4 | 61.0 | 91.7 | 73.2 |
| NPRBZ | 0 | 0 | .06 | .68 | 0 | .36 |
| Other | 2.1 | 3.1 | 5.2 | 12.9 | 4.5 | 8.5 |
| DIPB % | | | | | | |
| para | 77.6 | 77.7 | 54.3 | 32.8 | 72.2 | 37.5 |
| meta | 21.4 | 21.7 | 45.4 | 66.9 | 27.6 | 62.3 |
| ortho | 1.0 | .6 | .3 | .3 | .2 | .2 |

- 11 -

## EXAMPLE 3

The ZSM-12 catalyst employed in this Example was an HZSM-12 catalyst having a silica to alumina ratio of 180. The hydrogen form of the zeolite was prepared by calcination, $NH_4+$ ion exchange and final calcination. The catalyst was steamed for 7 hours at 1000°F (538°C), 100 percent steam and atmospheric pressure. The catalyst had an Alpha Value of 30.

Conditions of reaction and a summary of analytical results are shown in Table 3, wherein "CU" stands for cumene, "NPRBZ" stands for n-propylbenzene and "Other" represents primarily propylene oligomers.

## Table 3

### Alkylation of Cumene with Propylene to Produce Diisopropylbenzene
### (at atmospheric pressure)

| | 1 | 2 | 3 | 4 | 5 | 6 |
|---|---|---|---|---|---|---|
| Run No. | | | | | | |
| Temp., °C | 150 | 200 | 250 | 300 | 250 | 300 |
| $CU/C_3H_6$ | | | | | | |
| WHSV | 7.1/.85 | 7.1/.85 | 7.1/.85 | 7.1/.85 | 14.4/1.65 | 14.4/1.65 |
| Mole | 2.9/1 | 2.9/1 | 2.9/1 | 2.9/1 | 3.1/1 | 3.1/1 |
| **Conversion, wt%** | | | | | | |
| CU | 7.8 | 7.2 | 10.7 | 33.1 | 15.7 | 30.2 |
| $C_3H_6$ | 40.0 | 47.3 | 63.9 | 53.2 | 73.3 | 50.4 |
| **Selectivity, wt%** | | | | | | |
| Benzene | 3.0 | 1.3 | 3.2 | 27.6 | 1.6 | 14.9 |
| DIPB | | | | | | |
| para | 66.4 | 76.0 | 61.5 | 20.7 | 69.1 | 31.5 |
| meta | 20.0 | 16.8 | 26.4 | 36.3 | 24.6 | 45.2 |
| ortho | 3.9 | .9 | .3 | .3 | .2 | .3 |
| Total | 90.3 | 93.7 | 88.2 | 57.3 | 93.3 | 77.0 |
| NPRBZ | .01 | .01 | .01 | 0 | 0 | 0 |
| Other | 6.7 | 5.0 | 8.6 | 14.8 | 4.5 | 8.1 |
| DIPB % | | | | | | |
| para | 73.5 | 81.1 | 69.7 | 36.2 | 73.5 | 40.9 |
| meta | 22.1 | 17.9 | 29.9 | 63.3 | 26.2 | 58.8 |
| ortho | 4.4 | 1.0 | .4 | .5 | .3 | .3 |

0148584

## EXAMPLE 4

The ZSM-12 catalyst of this Example was a magnesium modified catalyst (i.e. Mg-ZSM-12) prepared by impregnating the unsteamed catalyst of Example 3 with aqueous magnesium nitrate. This impregnated catalyst was filtered, dried and calcined in air in a shallow dish in a furnace by slowly increasing the temperature from 30-500°C over 1.5 hours and standing overnight at 500°C. The resulting Mg-ZSM-12 contained 2.3 weight percent of Mg.

Conditions of reaction and a summary of analytical results are shown in Table 4, wherein "CU" stands for cumene, "NPRBZ" stands for n-propylbenzene and "Other" represents primarily propylene oligomers.

## Table 4

### Alkylation of Cumene with Propylene to Produce Diisopropylbenzene
### (at atmospheric pressure)

| Run No. | 1 | 2 | 3 | 4 | 5 |
|---|---|---|---|---|---|
| Temp., °C | 150 | 200 | 250 | 300 | 300 |
| $CU/C_3H_6$ | | | | | |
| WHSV | 7.2/.85 | 7.2/.85 | 7.2/.85 | 7.2/.85 | 7.2/.85 |
| Mole | 3.1 | 3.1 | 3.1 | 3.1 | 3.1 |
| **Conversion, wt%** | | | | | |
| CU | 4.4 | 4.2 | 10.4 | 23.6 | 29.2 |
| $C_3H_6$ | 8.3 | 9.0 | 68.7 | 42.0 | 46.6 |
| **Selectivity, wt%** | | | | | |
| Benzene | 7.6 | 2.6 | 2.9 | 23.1 | 10.2 |
| DIPB | | | | | |
| para | 70.9 | 77.5 | 68.3 | 28.2 | 35.3 |
| meta | 16.4 | 16.1 | 23.4 | 37.5 | 36.5 |
| ortho | .65 | .48 | .29 | .35 | .45 |
| Total | 88.0 | 94.1 | 92.0 | 66.1 | 72.3 |
| NPRBZ | .69 | .38 | .13 | 0 | 0 |
| Other | 3.7 | 2.9 | 5.0 | 10.8 | 17.5 |
| DIPB % | | | | | |
| para | 80.7 | 82.4 | 74.3 | 42.7 | 48.8 |
| meta | 18.6 | 17.1 | 25.4 | 56.8 | 50.5 |
| ortho | .7 | .5 | .3 | .5 | .6 |

## EXAMPLE 5

The ZSM-12 catalyst of this Example was a catalyst modified with both phosphorous and magnesium (i.e. P-Mg-ZSM-12) prepared by first treating the unsteamed catalyst of Example 3 with magnesium in accordance with the general procedure of Example 4 and then by treating with phosphorous. The phosphorous treating procedure was essentially equivalent to the magnesium treating procedure except that the catalyst was impregnated with aqueous diammonium acid phosphate (DAAP). The resulting P-Mg-ZSM-12 catalyst contained 5.4 weight percent P and 0.2 weight percent Mg.

Conditions of reaction and a summary of analytical results are shown in Table 5, wherein "CU" stands for cumene, "NPRBZ" stands for n-propylbenzene and "Other" represents primarily propylene oligomers.

## Table 5

### Alkylation of Cumene with Propylene to Produce Diisopropylbenzene
### (at atmospheric pressure)

| Run No. | 1 | 2 | 3 | 4 |
|---|---|---|---|---|
| Temp., °C | 150 | 200 | 250 | 300 |
| $CU/C_3H_6$ | | | | |
| WHSV | 3.4 | 3.4 | 3.4 | 3.4 |
| Mole | 3/1 | 3/1 | 3/1 | 3/1 |
| **Conversion, wt%** | | | | |
| CU | tr[a] | tr | tr | 4.0 |
| $C_3H_6$ | 28 | 29 | 27 | 15 |
| **Selectivity, wt%** | | | | |
| Benzene | 34.5 | 15.2 | 7.1 | 19.0 |
| DIPB | | | | |
| para | 23.8 | 52.6 | 66.1 | 54.4 |
| meta | 15.5 | 8.7 | 11.1 | 14.7 |
| ortho | 0 | 0 | 0 | .3 |
| Total | 39.3 | 61.3 | 77.2 | 69.4 |
| NPRBZ | 3.1 | 2.8 | .05 | .02 |
| Other | 23.1 | 20.7 | 15.6 | 11.6 |
| DIPB % | | | | |
| para | 60.5 | 85.8 | 85.7 | 78.3 |
| meta | 39.5 | 14.2 | 14.3 | 21.2 |
| ortho | 0 | 0 | 0 | .5 |

(a) Trace amounts detected.

## EXAMPLE 6

The ZSM-12 catalyst of this Example was a phosphorous modified catalyst (i.e. P-ZSM-12) prepared by impregnating the unsteamed catalyst of Example 3 with aqueous diammonium acid phosphate. This impregnated catalyst was filtered, dried and calcined in air in a shallow dish in a furnace by slowly increasing the temperature from 30-500°C over 1.5 hours and standing overnight at 500°C. The resulting P-ZSM-12 contained 2.69 weight percent of P.

Conditions of reaction and a summary of analytical results are shown in Table 6, wherein "CU" stands for cumene, "NPRBZ" stands for n-propylbenzene and "Other" represents primarily propylene oligomers.

## Table 6

### Alkylation of Cumene with Propylene to Produce Diisopropylbenzene
(at atmospheric pressure)

| Run No. | 1* | 2 | 3* | 4 | 5 | 6 |
|---|---|---|---|---|---|---|
| Temp., °C | 150 | 200 | 200 | 250 | 250 | 300 |
| $CU/C_3H_6$ | | | | | | |
| WHSV | 7.3/.83 | 7.3/.83 | 7.3/.85 | 7.3/.83 | 7.3/.85 | 7.3/.85 |
| Mole | 3.1/1 | 3.1/1 | 3.0/1 | 3.1/1 | 3.0/1 | 3.0/1 |
| **Conversion, wt%** | | | | | | |
| CU | .9 | 1.3 | 5.8 | 5.6 | 10.8 | 23.0 |
| $C_3H_6$ | 19.2 | 12.7 | 59.1 | 23.5 | 96.9 | 35.4 |
| **Selectivity, wt%** | | | | | | |
| Benzene | 0 | 19.0 | .5 | 4.7 | 3.0 | 23.0 |
| DIPB | | | | | | |
| para | 71.8 | 57.9 | 74.8 | 72.0 | 61.9 | 26.7 |
| meta | 21.6 | 13.1 | 19.5 | 19.6 | 29.6 | 34.2 |
| ortho | 3.2 | 0 | 1.3 | .5 | .5 | .3 |
| Total | 96.6 | 71.0 | 95.6 | 92.1 | 92.0 | 61.2 |
| NPRBZ | 0 | 1.21 | .15 | .31 | .10 | .57 |
| Other | 3.4 | 8.8 | 3.7 | 2.9 | 4.9 | 15.2 |
| DIPB % | | | | | | |
| para | 74.3 | 81.5 | 78.3 | 78.1 | 67.3 | 43.6 |
| meta | 22.4 | 18.5 | 20.4 | 21.3 | 32.2 | 55.9 |
| ortho | 3.3 | 0 | 1.3 | .6 | .5 | .5 |

*calcined before use

F-2628 (2629)                    -20-                    0148584

## EXAMPLE 7

The ZSM-12 catalyst of this Example was prepared in the same manner as the catalyst of Example 3 except that it was steamed for 3 hours instead of 7 hours.  The Alpha Value of this catalyst was not determined.

Conditions of reaction and a summary of analytical results are shown in Table 7, wherein "CU" stands for cumene, "NPRBZ" stands for n-propylbenzene and "Other" represents primarily propylene oligomers.

## Table 7

### Alkylation of Cumene with Propylene to Produce Diisopropylbenzene (at 500 psig [3549kPa])

| Run No. | 1 | 2 | 3 | 4 | 5* | 6 | 7 | 8 |
|---|---|---|---|---|---|---|---|---|
| Temperature, °C | 150 | 150 | 150 | 200 | 200 | 200 | 250 | 250 |
| $CU/C_3H_6$ | | | | | | | | |
| WHSV | 7.1/.84 | 7.1/.84 | 7.1/.84 | 7.1/.84 | 7.4/.83 | 13.8/.83 | 13.3/.83 | 7.3/.83 |
| mole | 2.9/1 | 2.9/1 | 2.9/1 | 2.9/1 | 3.1/1 | 5.8/1 | 5.8/1 | 3.1/1 |
| **Conversion, wt%** | | | | | | | | |
| CU | 11.1 | 10.5 | 8.2 | 17.9 | 9.1 | 11.0 | 19.1 | 32.2 |
| $C_3H_6$ | 96.5 | 71.8 | 46.8 | 88.8 | 100 | 97 | 99 | 99 |
| **Selectivity, %** | | | | | | | | |
| Benzene | .5 | 1.5 | .5 | .5 | .1 | .2 | 4.9 | 6.1 |
| DIPB | | | | | | | | |
| para | 66.4 | 61.9 | 60.3 | 65.8 | 68.9 | 71.8 | 57.3 | 42.5 |
| meta | 18.1 | 17.8 | 17.5 | 16.9 | 18.4 | 18.1 | 27.6 | 32.8 |
| ortho | 2.7 | 2.8 | 2.6 | 1.5 | 1.3 | 1.4 | .5 | .6 |
| Total | 87.2 | 82.5 | 80.4 | 84.2 | 88.6 | 91.3 | 85.4 | 77.9 |
| NPRBZ | .18 | .80 | .88 | .23 | .84 | .49 | .16 | .12 |
| Other | 12.1 | 15.2 | 18.2 | 15.1 | 10.5 | 18.10 | 9.5 | 15.8 |
| DIPB % | | | | | | | | |
| para | 76.2 | 75.1 | 74.9 | 78.2 | 77.7 | 78.7 | 67.1 | 54.5 |
| meta | 20.8 | 21.5 | 21.8 | 20.0 | 20.8 | 19.8 | 32.3 | 44.7 |
| ortho | 3.0 | 3.4 | 3.3 | 1.8 | 1.5 | 1.5 | 0.6 | .8 |

*Calcine 18 hrs. before making run.

With regard to the foregoing Examples, it is noted that the runs set forth in Example 7 were carried out at a pressure of 500 psig (3549kPa). The remainder were made at atmospheric pressure. The temperature range varied from 150-300°C. The weight hourly space velocity varied from about 8-15 and the molar cumene/propylene feed ratio varied from about 3/1 to 6/1.

In order to demonstrate the effect of pressure, data from Tables 1, 2 and 7 are summarized in Table 8. This data corresponds to the catalysts of Examples 1, 2 and 7, which are each similar but not identical acid forms of ZSM-12 (i.e. HZSM-12).

## Table 8

### Alkylation of Cumene with Propylene to Produce Diisopropylbenzene
### Effect of Pressure

| | | | | | | | | |
|---|---|---|---|---|---|---|---|---|
| Table No. | 7 | 2 | 7 | 2 | 1 | 7 | 2 | 1 |
| Run No. | 1 | 1 | 4 | 2 | 2 | 8 | 3 | 4 |
| Temperature, °C | 150 | 150 | 200 | 200 | 200 | 250 | 250 | 250 |
| Pressure, psig(kPa) | 500 (3549) | 0 (101) | 500 (3549) | 0 (101) | 0 (101) | 500 (3549) | 0 (101) | 0 (101) |
| $CU/C_3H_6$ | | | | | | | | |
|   WHSV | 7.1/.8 | 7.2/.9 | 7.2/.9 | 7.2/.9 | 7.6/.87 | 7.2/.9 | 7.2/.9 | 6.7/.87 |
|   mole | 2.9/1 | 2.9/1 | 2.9/1 | 2.9/1 | 3.1/1 | 2.9/1 | 2.9/1 | 2.7/1 |
| **Conversion, wt%** | | | | | | | | |
|   CU | 11.1 | 13.4 | 17.9 | 17.2 | 26 | 32.2 | 26.9 | 36 |
|   $C_3H_6$ | 96.5 | 27.5 | 88.8 | 48.0 | 70 | 99 | 76.7 | 89 |
| **Selectivity, %** | | | | | | | | |
|   Benzene | 5 | 11.0 | .5 | 1.5 | 1.2 | 6.1 | 4.3 | 3.3 |
|   DIPB | | | | | | | | |
|     para | 66.4 | 67.4 | 65.8 | 74.2 | 68.6 | 42.5 | 49.1 | 42.6 |
|     meta | 18.1 | 18.6 | 16.9 | 20.7 | 23.1 | 34.8 | 41.1 | 47.8 |
|     ortho | 2.7 | .9 | 1.5 | .5 | .9 | .6 | .2 | .3 |
|     Total | 87.2 | 86.9 | 84.2 | 95.4 | 92.6 | 77.9 | 90.0 | 90.7 |
|   NPRBZ | .18 | 0 | .23 | 0 | 0 | .12 | .06 | 0 |
|   Other | 12.1 | 2.1 | 15.1 | 3.1 | 6.2 | 15.8 | 5.2 | 6.0 |
|   DIPB % | | | | | | | | |
|     para | 76.2 | 77.6 | 78.2 | 77.7 | 74.1 | 54.5 | 54.3 | 47.0 |
|     meta | 20.8 | 21.4 | 20.0 | 21.7 | 29.4 | 44.7 | 45.4 | 52.7 |
|     ortho | 3.0 | 1.0 | 1.8 | .6 | 1.0 | .8 | .3 | .3 |

At 500 psig (3549kPa), Table 7, the reaction was primarily in liquid/solid heterogeneous phases. By contrast, at 0 psig (101 kPa) it was a heterogeneous gas/solid reaction, Tables 1 and 2. Examination of the data indicates that (a) cumene conversions were similar at 0 and 500 psig (101 and 3549 kPa), (b) propylene conversion was significantly higher at 500 psig (3549kPa), (c) the selectivity to DIPB was highest at 0 psig (101kPa), (d) less n-propylbenzene was made at 0 psig (101kPa), (e) signficantly less by-products were made at 0 psig (101kPa), and (f) less ortho-DIPB was made at 0 psig (101kPa).

In summary, better overall performance was observed at 0 psig (101kPa) compared with 500 psig (3549kPa). The para/meta ratio of DIPB was not influenced by pressure.

Data from the foregoing Examples also demonstrates the effect of temperature. Runs were made in the 150-300°C temperature range. As expected, conversion increased significantly with increases in temperature. Propylene is relatively reactive over the catalyst. Undesired by-products which consume this starting material are formed at the higher temperatures. By using higher cumene/propylene feed ratios (or a multiple bed reactor to achieve this effect), more efficient propylene use could be realized.

One of the primary effects of temperature is to change the para/meta isomer ratio of the DIPB product. At lower temperatures, 150-200°C, the para isomer is favored, p/m is about 78/20. At 250 and 300°C, the p/m ratios are 33-38/67-62.

Data from the foregoing Examples also demonstrates the effect of impregnations with aqueous solutions of magnesium or phosphorous salts followed by calcination. A catalyst was prepared which contained 2.3% magnesium, present as the oxide, on ZSM-12 and tested for preparation of DIPB. Results are summarized in Table 4. Relatively high amounts of para isomer in the DIPB product (81-82%) were observed. Similar results were observed with phosphorus (82% p-DIPB), Table 6, and a combination of Mg and P (86% p-DIPB), Table 5. Excellent selectivity to total DIPB was also observed with P-ZSM-12 and Mg-ZSM-12. As expected, cumene conversion was reduced by impregnation with Mg or P.

Perhaps other impregnation reagents with metal cations larger in size than Mg or P could achieve higher para-selectivity, e.g., to produce 90% or more para isomer in the DIPB product at practical conversions.

Data from the foregoing Examples also demonstrates the effect of steaming of the ZSM-12 catalyst. One sample of HZSM-12, Table 3, was steamed for 7 hours to an alpha = 30. Tables 1 and 2 relate to unmodified catalysts with a higher silica/alumina ratio (213/180). Except for somewhat lower cumene conversion, similar performance was observed for these three catalysts in all categories.

Data from the foregoing Examples also demonstrates how the selection of a particular ZSM-12 catalyst can effect the relative yield of diisopropylbenzene.

The para-diisopropylbenzene product of the foregoing Examples may be oxidized, e.g., with air, to the corresponding dihydroperoxide and rearranged by an acid catalyst to hydroquinone and acetone. The meta isomer is converted to resorcinol and acetone by an analogous process.

WHAT IS CLAIMED IS:

1.  A process for the selective production of
para-diisopropylbenzene by propylation of cumene, said process
comprising a contacting mixture of cumene and propylene with a
ZSM-12 catalyst under propylation conditions.

2.  A process for the selective production of
para-diisopropylbenzene, said process comprising the step of:

(i)  contacting mixtures of benzene and propylene with a
ZSM-12 catalyst under propylation conditions to selectively produce
cumene; and

(ii) contacting mixtures of said cumene of step (i) and
propylene with a ZSM-12 catalyst under propylation conditions.

3.  A process for the selective production of
para-diisopropylbenzene by propylation of benzene with said process
comprising contacting a mixture of benzene and propylene with a
ZSM-12 catalyst under propylation conditions, wherein the molar
ratio of propylene to benzene is at least 2.

4.  The process of any preceding Claim, wherein said
propylation conditions comprise a temperature of between 100°C and
300°C, and a pressure of between $10^5$ N/m$^2$ and 6 X $10^6$ N/m$^2$.

5.  The process of Claim 4 wherein said temperature is
between 150°C and 250°C.

6.  The process of Claim 1 or Claim 2 wherein the molar
ratio of cumene to propylene is within the range of 12 to 1.

F-2628 (2629)                    -27-                    0148584

7.  The process of any one of Claims 1, 2 and 6 wherein said cumene/propylene mixture is contacted with said catalyst as a continuous stream at a weight hourly space velocity of 0.05 to 100.

8.  The process of Claim 7 wherein said weight hourly space velocity is between 5 and 40.

9.  The process of any preceding Claim wherein the diisopropylbenzene produced combines at least 80% of the para isomer.

10.  A method of selectively producing hydroquinone comprising the steps of producing para-diisopropylbenzene by a process as claimed in any preceding Claim; oxidizing said para-diisopropylbenzene to the corresponding dihydroperoxide; and rearranging said dihydroperoxide to produce hydroquinone and acetone.

4896H/0299H